Europäisches Patentamt

European Patent Office

Office européen des brevets

(19)

(11) **EP 1 647 912 A2**

(12) **EUROPEAN PATENT APPLICATION**

(43) Date of publication:
**19.04.2006 Bulletin 2006/16**

(51) Int Cl.:
**G06F 19/00** (2006.01)

(21) Application number: **05256301.2**

(22) Date of filing: **10.10.2005**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **12.10.2004 US 964524
12.05.2005 US 128896**

(71) Applicant: **Agilent Technologies, Inc.
Palo Alto, CA 94306 (US)**

(72) Inventors:
• **Vallaya, Aditya
Loveland
Colorado 80537-0599 (US)**
• **Kincaid, Robert H.
Loveland
Colorado 80537-0599 (US)**

(74) Representative: **Tollett, Ian et al
Williams Powell
Morley House
26-30 Holborn Viaduct
London EC1A 2BP (GB)**

(54) **Methods and systems for ontological integration of disparate biological data**

(57) Methods, systems and computer readable media for correlating data from data sets to higher level categories of characterization of the data. Data from a first set of data (420,440,460) is analyzed to determine where members of the first set map to an ontology (400). Data from a second set of data (420,440,460) is analyzed to determine where members of the second set map to the ontology (400). From such analysis a subset of the first set of data (420,440,460) is identified and a subset of the second set of data (420,440,460) is identified. The subset of the first set of data (420,440,460) is statistically analyzed with regard to its mapping to the ontology (400), and a first set of ontology terms (410) are identified that are statistically differentiated by members of the subset of the first set of data (420,440,460). The subset of the second set of data (420,440,460) is statistically analyzed with regard to its mapping to the ontology (400), and a second set of ontology terms (410) is identified that are statistically differentiated by members of the subset of the second set of data (420,440,460). Correlation of the first set of ontology terms (410) with the second set of ontology terms (410) may further be performed.

FIG. 5

**Description**

The present invention relates to a method of and a system for correlating data to higher level categories of characterization of the data. The present invention further relates to a computer readable medium carrying one or more sequences of instructions for correlating the data.

Molecular biologists need to assimilate knowledge from a dramatically increasing amount and diversity of biological data. The advent of high-throughput experimental technologies for molecular biology have resulted in an explosion of data and a rapidly increasing variety of biological measurement data types. Examples of such biological measurement types include gene expression from DNA microarray or Quantitative PCR experiments, array CGH data based on CGH arrays, genotyping data based on microarrays, protein identification and abundance measurement from protein arrays, mass spectrometry or gel electrophoresis, metabolite identification and abundance using LC/MS, CE/MS, and mass spectrometry, etc.

In order to compare disparate data, researchers generally need a common identifier (often just a gene/protein symbol) in order to make a comparison between data types. However, this becomes difficult when different measurement platforms may riot have comparable probe sets. For example, mass spectra rarely coincide precisely with the content of a DNA microarray. It is even more difficult to compare metabolites with protein or gene expression data. In these instances, there is no connection between data types, such as the central dogma of expression/translation. However, the molecules are still related via some process or category, and it would be useful to identify some relationship for comparison.

A number of research groups have addressed the problem of identifying interesting pathways or GO (Gene Ontology) processes based on gene expression data. Such analyses can also be extended to high-throughput protein data, since genes and the corresponding proteins are directly related. However, metabolites are not directly related to genes or proteins via the "central dogma". Hence, experimental data representing abundance or presence of metabolites cannot be easily integrated with genomic or proteomic data.

Thus there is a continuing need for solutions for combining heterogeneous data from categories that are not typically directly related. What is needed are solutions for relying upon more indirect associations to combine data from various categories that may be related, although not directly related.

Methods, systems and computer readable media carrying for correlating data from data sets to higher level categories of characterization of the data. Data from a first set of data is analyzed to determine where members of the first set map to an ontology. Data from a second set of data is analyzed to determine where members of the second set map to the ontology. From such analysis a subset of the first set of data is identified and a subset of the second set of data is identified. The subset of the first set of data is statistically analyzed with regard to its mapping to the ontology, and a first set of ontology terms are identified that are statistically differentiated by members of the subset of the first set of data. The subset of the second set of data is statistically analyzed with regard to its mapping to the ontology, and a second set of ontology terms is identified that are statistically differentiated by members of the subset of the second set of data. Correlation of the first set of ontology terms with the second set of ontology terms may further be performed.

These and other advantages and features of the invention will become apparent to those persons skilled in the art upon reading the details of the methods, systems and computer readable media as more fully described below.

Preferred embodiments of the present invention will now be described, by way of example only, and with reference to the accompanying drawings, in which:

Fig. 1 shows an exemplary substrate carrying an array, such as may be used in the devices of the subject invention.
Fig. 2 shows an enlarged view of a portion of Fig. 1 showing spots or features.
Fig. 3 is an enlarged view of a portion of the substrate of Fig. 1.
Fig. 4A shows a schematic example of a portion of an ontology.
Figs. 4B, 4C and 4D schematically represent sets of data to be correlated using the ontology of Fig. 4A, respectively.
Fig. 5 is a flowchart of events that may be carried out in practicing an embodiment of the present invention.
Fig. 6A schematically illustrates a portion of an ontology in which the ontology terms may be networks or biological pathways and/or subnetworks or subpathways.
Fig. 6B further schematically illustrates an example of a biological pathway that may make up an ontology term in an ontology such as is illustrated in Fig. 6A.
Fig. 7 shows an example of an output produced on a display of a user interface wherein the abstraction of results in terms of ontology term membership, from sets of diverse experimental data studying a particular biological process or classification, are displayed.
Fig. 8 illustrates a typical computer system that may be used to practice an embodiment of the present invention.

Before the present methods and systems are described, it is to be understood that this invention is not limited to particular data, methods, hardware, software or algorithms described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a subset" includes a plurality of such subsets and reference to "the network diagram" includes reference to one or more network diagrams and equivalents thereof known to those skilled in the art, and so forth.

The publications discussed herein are provided solely for their disclosure prior to the filing date of the present application. Nothing herein is to be construed as an admission that the present invention is not entitled to antedate any such publication by virtue of prior invention. Further, the dates of publication provided may be different from the actual publication dates which may need to be independently confirmed.

## DEFINITIONS

The term "ontology" refers to an explicit formal specification of how to represent objects, concepts and/or other entities that are assumed to exist in some area of interest, and the relationships that hold among such objects, concepts and/or other entities. One non-limiting example of an ontology is a hierarchical structuring of knowledge about things by subcategorizing them according to their essential (or at least relevant and/or cognitive) qualities.

"Ontology terms" are terms that make up an ontology and which are used in the ontology in identifying the relationships referred to above. Ontology terms may include GO (gene ontology) terms, biological diagrams and subdiagrams, networks and subnetworks, cellular locations, concepts to disease association, concepts to drug compound association, etc, or any arbitrary grouping of concepts that may be deemed biologically interesting.

The term "oligomer" is used herein to indicate a chemical entity that contains a plurality of monomers. As used herein, the terms "oligomer" and "polymer" are used interchangeably. Examples of oligomers and polymers include polydeoxyribonucleotides (DNA), polyribonucleotides (RNA), other nucleic acids that are C-glycosides of a purine or pyrimidine base, polypeptides (proteins) or polysaccharides (starches, or polysugars), as well as other chemical entities that contain repeating units of like chemical structure.

"Disparate data" refers to data that reports measurements or characteristics of an object of study using different measurement criteria. In order to compare disparate data, researchers generally need a common identifier (often just a gene/protein symbol) in order to make a comparison between data types. However, this becomes difficult when different measurement platforms may not have comparable probe sets. Non-limiting examples of disparate data include metabolite data and gene expression data, as well as metabolite data and protein data. Disparate data, as described herein, while not directly related, are still related via some process or category.

The term "nucleic acid" as used herein means a polymer composed of nucleotides, e.g., deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length.

The term "functionalization" as used herein relates to modification of a solid substrate to provide a plurality of functional groups on the substrate surface. By a "functionalized surface" is meant a substrate surface that has been modified so that a plurality of functional groups are present thereon.

The terms "reactive site", "reactive functional group" or "reactive group" refer to moieties on a monomer, polymer or substrate surface that may be used as the starting point in a synthetic organic process. This is contrasted to "inert" hydrophilic groups that could also be present on a substrate surface, e.g., hydrophilic sites associated with polyethylene glycol, a polyamide or the like.

The term "sample" as used herein relates to a material or mixture of materials, typically, although not necessarily, in fluid form, containing one or more components of interest.

The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The phrase "oligonucleotide bound to a surface of a solid support" refers to an oligonucleotide or mimetic thereof, e.g., PNA, that is immobilized on a surface of a solid substrate in a feature or spot, where the substrate can have a variety of configurations, e.g., a sheet, bead, or other structure. In certain embodiments, the collections of features of oligonucleotides employed herein are present on a surface of the same planar support, e.g., in the form of an array.

The term "array" encompasses the term "microarray" and refers to an ordered array presented for binding to nucleic acids and the like. Arrays, as described in greater detail below, are generally made up of a plurality of distinct or different features. The term "feature" is used interchangeably herein with the terms: "features," "feature elements," "spots," "addressable regions," "regions of different moieties," "surface or substrate immobilized elements" and "array elements," where each feature is made up of oligonucleotides bound to a surface of a solid support, also referred to as substrate immobilized nucleic acids.

An "array," includes any one-dimensional, two-dimensional or substantially two-dimensional (as well as a three-dimensional) arrangement of addressable regions (i.e., features, e.g., in the form of spots) bearing nucleic acids, particularly oligonucleotides or synthetic mimetics thereof (i.e., the oligonucleotides defined above), and the like. Where the arrays are arrays of nucleic acids, the nucleic acids may be adsorbed, physisorbed, chemisorbed, or covalently attached to the arrays at any point or points along the nucleic acid chain.

Any given substrate may carry one, two, four or more arrays disposed on a front surface of the substrate. Depending upon the use, any or all of the arrays may be the same or different from one another and each may contain multiple spots or features. A typical array may contain one or more, including more than two, more than ten, more than one hundred, more than one thousand, more ten thousand features, or even more than one hundred thousand features, in an area of less than 20 cm$^2$ or even less than 10 cm$^2$, e.g., less than about 5 cm$^2$, including less than about 1 cm$^2$, less than about 1 mm$^2$, e.g., 100 $\mu^2$, or even smaller. For example, features may have widths (that is, diameter, for a round spot) in the range from a 10 $\mu$m to 1.0 cm. In other embodiments each feature may have a width in the range of 1.0 $\mu$m to 1.0 mm, usually 5.0 $\mu$m to 500 $\mu$m, and more usually 10 $\mu$m to 200 $\mu$m. Non-round features may have area ranges equivalent to that of circular features with the foregoing width (diameter) ranges. At least some, or all, of the features are of different compositions (for example, when any repeats of each feature composition are excluded the remaining features may account for at least 5%, 10%, 20%, 50%, 95%, 99% or 100% of the total number of features). Inter-feature areas will typically (but not essentially) be present which do not carry any nucleic acids (or other biopolymer or chemical moiety of a type of which the features are composed). Such inter-feature areas typically will be present where the arrays are formed by processes involving drop deposition of reagents but may not be present when, for example, photolithographic array fabrication processes are used. It will be appreciated though, that the inter-feature areas, when present, could be of various sizes and configurations.

Each array may cover an area of less than 200 cm$^2$, or even less than 50 cm$^2$, 5 cm$^2$, 1 cm$^2$, 0.5 cm$^2$, or 0.1 cm$^2$. In certain embodiments, the substrate carrying the one or more arrays will be shaped generally as a rectangular solid (although other shapes are possible), having a length of more than 4 mm and less than 150 mm, usually more than 4 mm and less than 80 mm, more usually less than 20 mm; a width of more than 4 mm and less than 150 mm, usually less than 80 mm and more usually less than 20 mm; and a thickness of more than 0.01 mm and less than 5.0 mm, usually more than 0.1 mm and less than 2 mm and more usually more than 0.2 and less than 1.5 mm, such as more than about 0.8 mm and less than about 1.2 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, the substrate may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulse-jets of either nucleic acid precursor units (such as monomers) in the case of in situ fabrication, or the previously obtained nucleic acid. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein. As already mentioned, these references are incorporated herein by reference. Other drop deposition methods can be used for fabrication, as previously described herein. Also, instead of drop deposition methods, photolithographic array fabrication methods may be used. Inter-feature areas need not be present particularly when the arrays are made by photolithographic methods as described in those patents.

In certain embodiments of particular interest, in situ prepared arrays are employed. In situ prepared oligonucleotide arrays, e.g., nucleic acid arrays, may be characterized by having surface properties of the substrate that differ significantly between the feature and inter-feature areas. Specifically, such arrays may have high surface energy, hydrophilic features and hydrophobic, low surface energy hydrophobic interfeature regions. Whether a given region, e.g., feature or interfeature region, of a substrate has a high or low surface energy can be readily determined by determining the regions "contact angle" with water, as known in the art and further described in co-pending application serial no. 10/449,838, the disclosure of which is herein incorporated by reference. Other features of in situ prepared arrays that make such array formats of particular interest in certain embodiments of the present invention include, but are not limited to: feature density, oligonucleotide density within each feature, feature uniformity, low intra-feature background, low inter-feature background, e.g., due to hydrophobic interfeature regions, fidelity of oligonucleotide elements making up the individual features, array/feature reproducibility, and the like. The above benefits of in situ produced arrays assist in maintaining adequate sensitivity while operating under stringency conditions required to accommodate highly complex samples.

An array is "addressable" when it has multiple regions of different moieties, i.e., features (e.g., each made up of different oligonucleotide sequences) such that a region (i.e., a "feature" or "spot" of the array) at a particular predetermined location (i.e., an "address") on the array will detect a particular solution phase nucleic acid sequence. Array features are typically, but need not be, separated by intervening spaces.

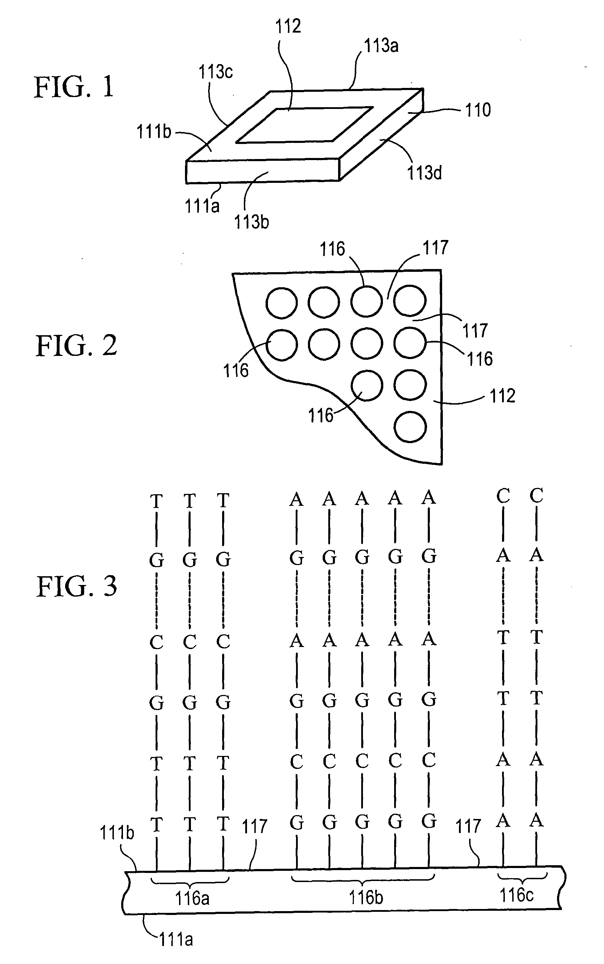

An exemplary array is shown in Figs. 1-3, where the array shown in this representative embodiment includes a contiguous planar substrate 110 carrying an array 112 disposed on a rear surface 111b of substrate 110. It will be appreciated though, that more than one array (any of which are the same or different) may be present on rear surface 111b, with or without spacing between such arrays. That is, any given substrate may carry one, two, four or more arrays disposed on a front surface of the substrate and depending on the use of the array, any or all of the arrays may be the same or different from one another and each may contain multiple spots or features. The one or more arrays 112 usually cover only a portion of the rear surface 111b, with regions of the rear surface 111b adjacent the opposed sides 113c, 113d and leading end 113a and trailing end 113b of slide 110, not being covered by any array 112. A front surface 111a of the slide 110 does not carry any arrays 112. Each array 112 can be designed for testing against any type of sample, whether a trial sample, reference sample, a combination of them, or a known mixture of biopolymers such as polynucleotides. Substrate 110 may be of any shape, as mentioned above.

As mentioned above, array 112 contains multiple spots or features 116 of oligomers, e.g., in the form of polynucleotides, and specifically oligonucleotides. As mentioned above, all of the features 116 may be different, or some or all could be the same. The interfeature areas 117 could be of various sizes and configurations. Each feature carries a predetermined oligomer such as a predetermined polynucleotide (which includes the possibility of mixtures of polynucleotides). It will be understood that there may be a linker molecule (not shown) of any known types between the rear surface 111b and the first nucleotide.

Substrate 110 may carry on front surface 111a, an identification code, e.g., in the form of bar code (not shown) or the like printed on a substrate in the form of a paper label attached by adhesive or any convenient means. The identification code contains information relating to array 112, where such information may include, but is not limited to, an identification of array 112, i.e., layout information relating to the array(s), etc.

In the case of an array in the context of the present application, the "target" may be referenced as a moiety in a mobile phase (typically fluid), to be detected by "probes" which are bound to the substrate at the various regions.

A "scan region" refers to a contiguous (preferably, rectangular) area in which the array spots or features of interest, as defined above, are found or detected. Where fluorescent labels are employed, the scan region is that portion of the total area illuminated from which the resulting fluorescence is detected and recorded. Where other detection protocols are employed, the scan region is that portion of the total area queried from which resulting signal is detected and recorded. For the purposes of this invention and with respect to fluorescent detection embodiments, the scan region includes the entire area of the slide scanned in each pass of the lens, between the first feature of interest, and the last feature of interest, even if there exist intervening areas that lack features of interest.

An "array layout" refers to one or more characteristics of the features, such as feature positioning on the substrate, one or more feature dimensions, and an indication of a moiety at a given location. "Hybridizing" and "binding", with respect to nucleic acids, are used interchangeably.

By "remote location," it is meant a location other than the location at which the array is present and hybridization occurs. For example, a remote location could be another location (e.g., office, lab, etc.) in the same city, another location in a different city, another location in a different state, another location in a different country, etc. As such, when one item is indicated as being "remote" from another, what is meant is that the two items are at least in different rooms or different buildings, and may be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. An array "package" may be the array plus only a substrate on which the array is deposited, although the package may include other features (such as a housing with a chamber). A "chamber" references an enclosed volume (although a chamber may be accessible through one or more ports). It will also be appreciated that throughout the present application, that words such as "top," "upper," and "lower" are used in a relative sense only.

The term "stringent assay conditions" as used herein refers to conditions that are compatible to produce binding pairs of nucleic acids, e.g., surface bound and solution phase nucleic acids, of sufficient complementarity to provide for the desired level of specificity in the assay while being less compatible to the formation of binding pairs between binding members of insufficient complementarity to provide for the, desired specificity. Stringent assay conditions are the summation or combination (totality) of both hybridization and wash conditions.

A "stringent hybridization" and "stringent hybridization wash conditions" in the context of nucleic acid hybridization (e.g., as in array, Southern or Northern hybridizations) are sequence dependent, and are different under different experimental parameters. Stringent hybridization conditions that can be used to identify nucleic acids within the scope of the invention can include, e.g., hybridization in a buffer comprising 50% formamide, 5×SSC, and 1% SDS at 42°C, or hybridization in a buffer comprising 5×SSC and 1% SDS at 65°C, both with a wash of 0.2×SSC and 0.1% SDS at 65°C. Exemplary stringent hybridization conditions can also include a hybridization in a buffer of 40% formamide, 1 M NaCl, and 1% SDS at 37°C, and a wash in 1×SSC at 45°C. Alternatively, hybridization to filter-bound DNA in 0.5 M NaHPO$_4$, 7% sodium dodecyl sulfate (SDS), 1 mM EDTA at 65°C, and washing in 0.1×SSC/0.1% SDS at 68°C can be employed. Yet additional stringent hybridization conditions include hybridization at 60°C or higher and 3 × SSC (450 mM sodium chloride/45 mM sodium citrate) or incubation at 42°C in a solution containing 30% formamide, 1M NaCl, 0.5% sodium sarcosine, 50 mM MES, pH 6.5. Those of ordinary skill will readily recognize that alternative but comparable hybridization and wash conditions can be utilized to provide conditions of similar stringency.

In certain embodiments, the stringency of the wash conditions that set forth the conditions which determine whether a nucleic acid is specifically hybridized to a surface bound nucleic acid. Wash conditions used to identify nucleic acids may include, e.g.: a salt concentration of about 0.02 molar at pH 7 and a temperature of at least about 50°C or about 55°C to about 60°C; or, a salt concentration of about 0.15 M NaCl at 72°C for about 15 minutes; or, a salt concentration of about 0.2xSSC at a temperature of at least about 50°C or about 55°C to about 60°C for about 15 to about 20 minutes; or, the hybridization complex is washed twice with a solution with a salt concentration of about 2×SSC containing 0.1% SDS at room temperature for 15 minutes and then washed twice by 0.1×SSC containing 0.1% SDS at 68°C for 15 minutes; or, equivalent conditions. Stringent conditions for washing can also be, e.g., 0.2×SSC/0.1% SDS at 42°C.

A specific example of stringent assay conditions is rotating hybridization at 65°C in a salt based hybridization buffer with a total monovalent cation concentration of 1.5 M (e.g., as described in U.S. Patent Application No. 09/655,482 filed on September 5, 2000, the disclosure of which is herein incorporated by reference) followed by washes of 0.5X SSC and 0.1X SSC at room temperature.

Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may also be employed, as appropriate.

Sensitivity is a term used to refer to the ability of a given assay to detect a given analyte in a sample, e.g., a nucleic acid species of interest. For example, an assay has high sensitivity if it can detect a small concentration of analyte molecules in sample. Conversely, a given assay has low sensitivity if it only detects a large concentration of analyte molecules (i.e., specific solution phase nucleic acids of interest) in sample. A given assay's sensitivity is dependent on a number of parameters, including specificity of the reagents employed (e.g., types of labels, types of binding molecules, etc.), assay conditions employed, detection protocols employed, and the like. In the context of array hybridization assays, such as those of the present invention, sensitivity of a given assay may be dependent upon one or more of: the nature of the surface immobilized nucleic acids, the nature of the hybridization and wash conditions, the nature of the labeling system, the nature of the detection system, etc.

Liquid chromatography/mass spectrometry (LC/MS) is a widely used technique for the global identification and quantitation of proteins, peptides and/or metabolites in complex biological samples. In this technique, liquid chromatography is used in-line with a mass spectrometer to chromatographically separate components prior to mass detection, in order to reduce the number of components presented to the mass spectrometer at a given time.

Liquid chromatography is an analytical chromatographic technique that is useful for separating components, typically ions or molecules, that are dissolved in a solvent. In this technique, the components (e.g., analytes) are first dissolved in a solvent and then are forced to flow through a chromatographic column that can range from a few centimeters to several meters. The column is packed with a solid phase chromatographic material that is matched to the solvents in use and binds the analytes via adsorption. An additional, different solvent is then mixed into the flow in increasing concentrations (such as by a smooth gradient increases, or step-wise increases, for example). Each compound in the analyte releases from the solid phase at a specific concentration of the additional solvent and then flows off of the column resulting in a serial separation of the compounds contained in the analyte. A variety of detectors for identifying the presence of compounds in the effluent have been developed over the past thirty years based on a variety of different sensing principles. Typically, signal intensity from a chromatographic detector can be plotted as a function of elution time (a chromatogram) and peaks are used to identify the components. Other techniques, such as characteristic retention time in a chromatographic column, may also be applied to identify the components. A mass spectrometer in this application functions as a very sensitive, multiplexed detector that can detect the presence of multiple compounds simultaneously and can differentiate between the compounds detected.

The evolution of mass spectrometry has been marked by an ever-increasing demand for improved sensitivity, resolution and mass accuracy and a wide variety of different techniques have been used to obtain them. However, at one level, the basic components of all mass spectrometers are essentially the same. These components may be best understood by tracing the ion's path through them. First, an ion source converts the analyte from the liquid (or solid) phase into the gas phase and places a charge on the molecules of the analyte. A common example of an ion source in an LC/MS system is electrospray ionization where the liquid phase input is sprayed into a chamber through a charged needle. Charge is deposited on the surface of the spray droplets and is transferred to the molecules of the analyte during the desolvation process where the solvents are evaporated off. Next, a mass analyzer differentiates the ions according to their mass-to-charge (m/z) ratio. Then, a detector measures the ion beam current to yield an m/z spectrum, where the peaks in the m/z spectrum may be used to differentiate and identify the input components.

2D Gels combined with mass spectrometry, usually MALDI-TOF, allow detection and identification of a large number of proteins from a tissue, and the comparison of protein profiles in different tissues, different genotypes or after different treatments. In addition to protein identification, 2D gel technology can be combined with the use of radiolabelling of the tissue before extraction, and subsequent autoradiography. Incubation with $^{32}$P will label proteins that are subject post-translational regulation by phosphorylation.

A general purpose method of metabolite assessment/quantitation does not exist, as no general characteristics account equally for all metabolites, given their differences in size, number and nature of functional groups, volatility, charge states or electromobility, polarity and other physicochemical parameters, see Fiehn et al., "Deciphering metabolic networks" http://content.febsjournal.org/cgi/content/full/270/4/579, 2003, pp 1-18, which is incorporated herein, in its entirety, by reference thereto. Moreover, each analytical detection method itself has a certain bias. For example, using mass spectrometry requires that metabolites are ionizable, coulometry needs analyte responses to varying redox potentials, ultraviolet absorption or fluorescence emission presumes that biochemical compounds bear moieties with excitable electrons (such as found in aromatic rings), and most other techniques are either too special (such as radioactivity detection), too insensitive (such as light scattering) or too difficult to be coupled to on-line separations (such as infrared spectroscopy). Therefore, no single metabolomic technique exists but a combination of aforementioned methods needs to be used.

The largest scope with respect to universality, sensitivity and selectivity is clearly achieved using mass spectrometry (MS). Applying different ionization techniques has proven very appropriate to detect a large variety of metabolites. For example, simple terpenes, carotenoids, or aliphatics are hardly chargeable by electrospray ionization (ESI), the standard technique used in conjunction with liquid chromatography (LC). Such hydrocarbons, however, are often volatile and can therefore easily be detected by a combination of gas chromatography (GC) and MS, for example using classical electron impact ionization. Thus, a combination of GC/MS and LC/MS methods is typically used for analyzing a wide range of metabolites.

**DESCRIPTION OF SPECIFIC EMBODIMENTS**

The present systems and methods make use of a common ontology between disparate data types to perform a statistical analysis yielding a higher level relationship among the data in the disparate data types, i.e., wherein the disparate data is not related among types on a one-to-one basis, but is categorically related among some higher level characterization (e.g., a process, network, classification, etc.) of that data that belongs to the higher level characterization and is identified as such. Computed association network, or other derived relationships between data can be generalized as a special ontology, which may even be user defined. While many of the examples herein rely upon Gene Ontology (http://www.geneontology.org), biological pathways and networks as examples of ontologies that may be used in carrying out the invention, it should be noted that the invention is not limited to these ontologies, as any suitable classification scheme that could be used for comparing the data at hand may serve as the ontology for purposes of the invention. For example, while genes, proteins and other molecules may be related using a biological pathway or network, other comparisons are possible, such as by defining an ontology based upon categorical terms such as cellular location, disease association, etc.

For examples where biological pathways and/or networks are used to define an ontology, pathway or network analysis is typically done by comparing data that qualifies as having pathway or network membership. However, the same type of analysis described herein may be carried out with regard to any ontology, e.g., any categories in which the data can be binned.

The discovery of medicines and treatments for various diseases is often a process of piecing together a detailed understanding of the molecular basis of disease in terms of articulating the story of how genes, proteins, and other small molecules interact with each other in biological networks. By understanding the structure and behavior of biological networks, i.e. the elements of the networks and the complex sets of interactions between them, biomedical researchers can identify intervention points for drugs and therapeutics, limit adverse side-effects of treatments, and infer predisposition to disease.

Biologists use experimental data, control data and numerous other sotirces of information to piece together interpretations and form hypotheses about biological processes. Such interpretations and hypotheses constitute higher-level models of biological activity. Such models can be the basis of communicating information to colleagues, for generating ideas for further experimentation, and for predicting biological response to a condition, treatment, or stimulus. Frequently these models take the form of biological networks and can be represented by network diagrams.

The present invention includes systems and methods for integrating diverse data types, based on ontological mapping, to determine a relationship among the diverse data at a level that is higher than a one-to-one correlation among the data members between the diverse data types. These systems and methods are particularly well suited for integrating data from diverse experimental data sets in terms, but are useful for any types of data that may be mapped to an ontology, as the ontology is used as a basis for formulating a relationship between the diverse types of data.

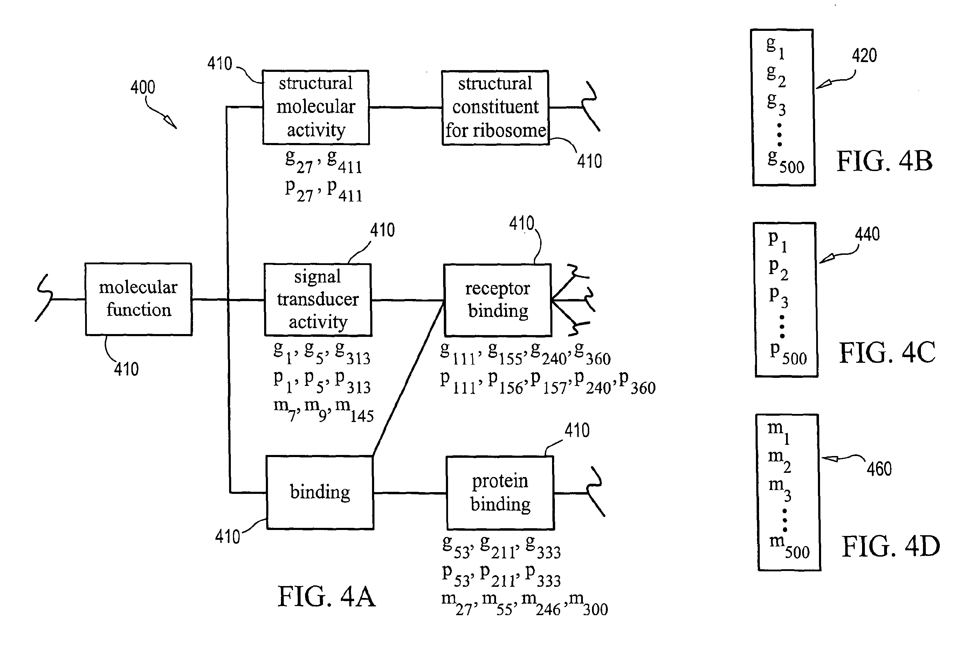

Referring now to Fig. 4A, a schematic example of a portion of an ontology 400 is displayed. In this example, the ontology from which the portion is displayed is the gene ontology (GO), but it is again emphasized here, that such is merely an example of an ontology that may be used and that the invention is in no way limited to using only gene ontology as a basis for determining correlations. In furtherance of this non-limiting example, sets of data 420, 460 and 480 are schematically represented at Figs. 4B, 4C and 4D, respectively. In this example, data set 420 includes a list of experimental data describing genes, such as expression data, for example. Data set 440 includes a list of protein abundance data, and data set 460 includes a list of data characterizing metabolites. The gene data, protein abundance data and metabolite data in data sets 420, 440 and 460 are all independent data. However for the integration of such data as described herein, the experiments measuring these different types of independent data will have been conducted on the same biological samples (i.e., on the same tissue or cell cultures, or the like).

It should be further noted that, for simplicity of the drawing and simplicity of explanation, the data sets are shown to be much smaller than what is normally encountered. Although the present invention is useable with data sets of the sizes shown, it is also very powerful for use with high throughput data, which typically produces much larger datasets. For example, a single microarray experiment producing data of the type described with regard to list 420 may produce twenty thousand entries, or more. Corresponding to such an expression data list, protein abundance entries may be in the thousands, or greater, and a corresponding metabolites list may contain hundreds to thousands of members. Metabolites may be measured according to their simple presence or absence in an experiment, or as to abundance, for example, using LC/MS, CE/MS, GC/MS, mass spectrometry, or the like, for example.

In order to determine some type of relationship among disparate data types, the members of the sets of disparate data must be mappable to a common ontology. In the example shown, members of each of data sets 420, 440 and 460 are mapped to the gene ontology 400. Again, only a few of the data are actually shown as mapped to the ontology terms 410 for simplicity, in order to meet drawing requirements. No data values have to actually be displayed as mapped at this stage, as long as they are mapped or mappable. Although not all gene, protein and metabolite data points need be mappable from the data sets 420, 440, 460 to the ontology terms 410, the closer to complete mapping that is achieved, the better are the results obtained from statistical analysis as described herein. That being said, those ontology terms 410 shown in Fig. 4A without any associated data from the sets 420,440,460 are not intended to convey that no data from the data sets are associated with those terms 410, but are simply not shown as such is unnecessary for purposes of the description.

It is further noted that, although gene expression data 420 and protein abundance data 440 are typically mappable to one another at the data level (e.g., a one-to-one, nearly one-to-one, or at least identifiable many-to-one or one-to-many specific mappings) and as such, are not typically referred to as "disparate data types", they can still be processed for determining one or more higher level associations according to the present methods. However, the metabolite data 460 in this example are considered to be disparate data with respect to the protein abundance data 440, as well as with respect to the gene expression data 420, are not directly related to genes or proteins via the "central dogma", as noted above. Hence, experimental data representing abundance or presence of metabolites cannot be easily integrated with genomic or proteomic data. Accordingly, the present methods are very powerful for use with disparate data in that disparate data may be integrated in terms of higher level classification, categorization or other description. Thus, for example associations between members of data set 460 may be made with members of data set 440 and/or 420.

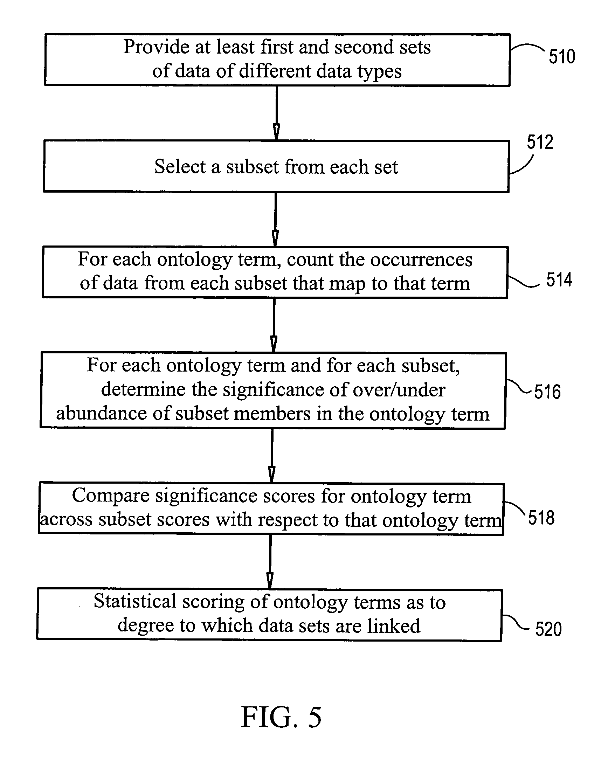

Still further, it is noted here that although the description of Figs. 4A-4D is with regard to three sets of data, that the present invention is not limited to this number or this combination of the types of data sets. For example, only two data types may be considered and processed, or more than three data types may be considered and processed (e.g., see Fig. 5, event 510). Further, although the invention is most powerful for integrating disparate data types, as noted, it is not strictly limited to such, as, for example, processing may be carried out with regard to data sets 420 and 440 without considering data set 460. Further, it is again stressed that the invention is not limited to the types of data represented in Figs. 4A-4D, or even to biological data. Rather, any data that are related via ontology term membership can be combined according to the techniques described herein.

Referring again to the example, one approach to combining the data types described is to first select a subset (event 512) of each of the data sets 420,440,460 that is of interest to the researcher or person running the analysis. For example, for biological experimentation such as may be described by data sets 420, 440 and 460, it is common to have at least one experimental sample and at least one control sample from which the data sets are generated, in order to be able to compare results in an effort to identify causations, explanations, etc. as to why the experimental sample(s) varies from the control sample(s). For example, an experimental sample may be cancer tissue, while the control sample is normal or non-cancerous tissue. In such a case, a subset of interest from data set 420 may be the set of genes that greatest differentiates the control and experimental samples, for example where gene expression is relatively very high in the experimental sample and relatively very low in the control sample, or where gene expression is relatively very low in the experimental sample and relatively very high in the control sample, or both., Similar types of sorting may be conducted for the protein abundance data set 440 and metabolites abundance data set 460.

Sorting of a data set can be accomplished in many ways and may vary according to the interests of the researcher or other person performing the analysis. For array data, such as data taken from microarrays or other tabular data, sorting may be performed using systems and tools as described in co-pending Application Serial Nos. 10/403,762 filed March 31, 2003 and titled "Methods and System for Simultaneous Visualization and Manipulation of Multiple Data Types" and 10/688,588 filed October 18, 2003, and titled "Methods and System for Simultaneous Visualization and Manipulation of Multiple Data Types", and in Kincaid, "VistaClara: an interactive visualization for exploratory analysis of DNA microarrays", Proceedings of the 2004 ACM symposium on Applied computing, ACM Press, 2004, pp 167-174, each of which are hereby incorporated herein, in their entireties, by reference thereto.

The selection of significant molecules can be based on a similarity searching of certain profiles, or on more robust statistical tests, for example. For simplicity of explanation, consider an experiment with two conditions, i.e., an experimental condition and a control condition. The approach to selection in this case is to identify a subset of molecules that differentiate the experimental condition from the control condition. An interesting pattern profile may be constructed by selecting molecules that meet certain conditions. For example an interesting pattern profile may be constructed by selecting molecules, the experimental values for which are high for the experimental condition and low for the control condition. That is, all molecules having expression/abundance values that are similar to the interesting pattern profile (up to a threshold value, which may be preset) are selected as members of the subset.

More robust statistical tests, such as t-test may be conducted to extract a subset of molecules that differentiate between two conditions. One example of a more robust test that may be used is SAM analysis, as described in Tusher et al., "Significance analysis of microarrays applied to the ionizing radiation response", PNAS 2001 98: 5116-5121, which is hereby incorporated herein, in its entirety, by reference thereto. Once a subset of interesting molecules has been identified, ontology terms can next be analyzed for over or under-representation with regard to the subset.

For each term in an ontology, data values that are members of the selected subset and that map to that ontology term are counted (event 514). Then an over or under abundance of the data values from the selected subset that occur within an ontology term may be calculated. For example, a Z-score may be calculated to measure the significance of the over/under abundance of an ontology term, given a selected subset, according to the following:

$$Z(ot) = \frac{(r - n\frac{R}{N})}{\sqrt{n(\frac{R}{N})(1 - \frac{R}{N})(1 - \frac{n-1}{N-1})}} \qquad (2)$$

where

Z(ot) = the Z-score with respect to the particular ontology term and the subset of data values being considered;

r = the number of entries (data values from subset) that map to *ot,*
n = the total number of data values in the subset
R = the number of entries(data values) in the full data set that map to *ot*, and
N = the total number of data values in the full data set.

For each subset (e.g., for the subsets from each of datasets 420, 440 and 460 in the example described with regard to Figs. 4A-4D above), significance values may be calculated with respect to each ontology term. After completion of such calculations, the significance scores of each ontology term may be compared across all subset scores at event 518. The results of such comparisons may be further processed at event 520 to provide some scoring scheme to indicate particular ontology terms that receive significant scores across all the data types considered. Such significant scoring ontology terms not only bridge the different data sets, but may also provide users with a level of abstraction above individual sets of molecules characterized by biologically meaningful and better understood ontological terms. For example, lists of genes, proteins and metabolites that significantly differentiate an experimental condition from the control may not be as meaningful to biologists as the ontology term "mitochondrion" or "fatty acid metabolism", which can be used to signify that all the significantly differentiating molecules are found in the mitochrondrion or participate in fatty acid metabolism, respectively. The system thus facilitates automatic identification of significant ontology terms from lists of significant molecules.

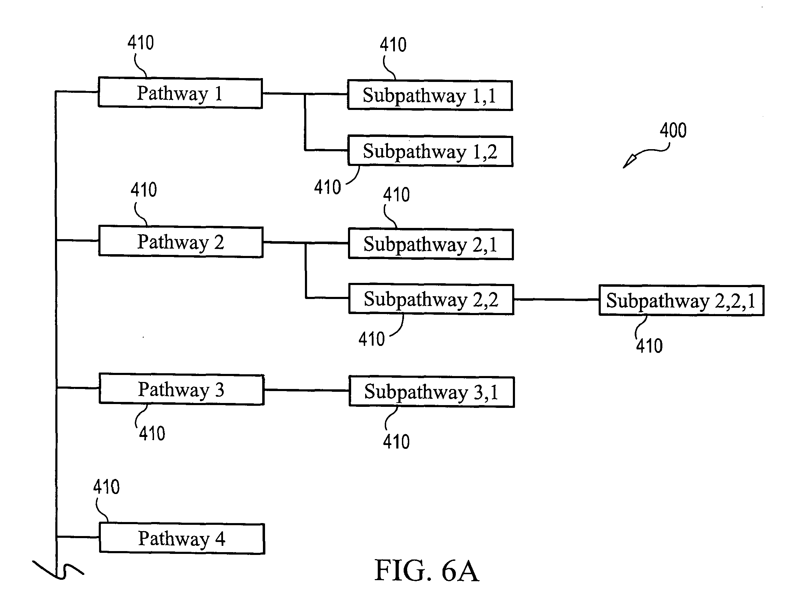

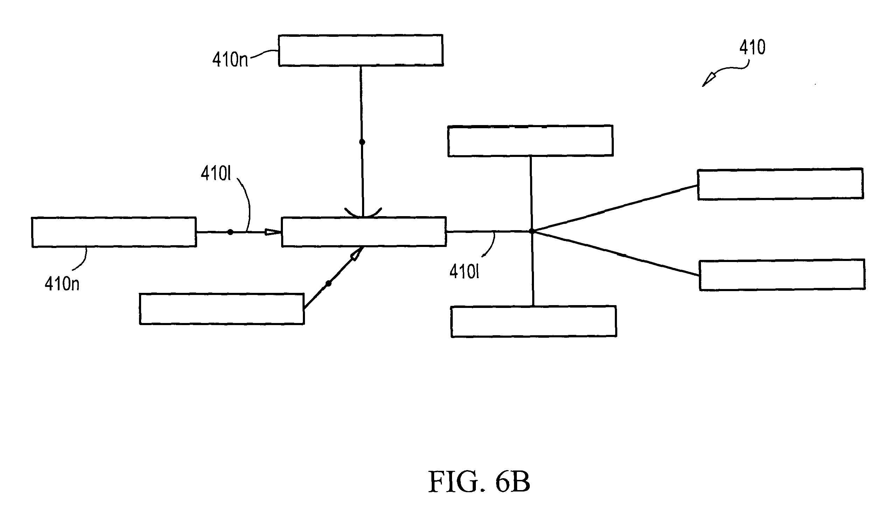

Fig. 6A schematically illustrates a portion of another ontology 400 in which the ontology terms in this example may be networks or biological pathways and/or subnetworks or subpathways, all of which are schematically illustrated as ontology terms 410 in Fig. 6A. Fig. 6B further schematically illustrates an example of a biological pathway that may make up an ontology term 410 in an ontology such as is illustrated in Fig. 6A. A biological pathway may include nodes 410n and links 410l used to describe a biological process by describing interaction (via links 410l) of entities (represented by nodes 410n). Such a pathway may represent relations between various entities that may also be represented by diverse data types, for example, genes, proteins, metabolites, other molecules, etc.

The networked graph data structures of the pathways 410 may be represented in terms of a local format that serves as a common representation for various qualitative models of biological processes, such as protein-protein interactions, metabolic and signal transduction pathways, regulatory networks, network representation of disease processes, etc. Further detailed description regarding the local format and its uses can be found in co-pending Application Serial Nos. 10/794,341 filed March 4, 2004 and titled "Methods and Systems for Extension , Exploration , Refinement and Analysis of Biological Networks; 10/155,675 filed May 22, 2002 and titled "System and Methods for Extracting Semantics from Images"; 10/641,492 filed August 14, 2003 and titled "Method and System for Importing, Creating and/or Manipulating Biological Diagrams"; 10/155,304 filed May 22, 2002 and titled "System, Tools and Methods to Facilitate Identification and Organization of New Information Based on Context of User's Existing Information"; 10/155,675 filed May 22, 2002 and titled "System and Methods for Extracting Semantics from Images"; 10/155,616 filed May 22, 2002 and titled "System and Methods for Visualizing Diverse Biological Relationships"; 10/154,524 filed May 22, 2002 and titled "System and-Method for Extracting Pre-Existing Data from Multiple Formats and Representing Data in a Common Format for Making Overlays"; 10/642,376 filed August 14, 2003 and titled "System, Tools and Method for Viewing Textual Documents, Extracting Knowledge Therefrom and Converting the Knowledge into Other Forms of Representation"; and 10/784,523 filed February 23, 2004 and titled "System, Tools and Method for Constructing Interactive Biological Diagrams"; each of which is hereby incorporated herein, in its entirety, by reference thereto.

The system assumes that there exists some mapping from the various concepts (e.g., data sets 420, 440, 460) to the pathway/network models 410, e.g., in terms of pathway information. Such information connecting these various concepts can be extracted from various public and proprietary life science databases including LocusLink, KEGG, BioCarta, Boehringer Mannheim metabolic pathway maps, BIND, DIP, etc. Such information can also be extracted from literature databases using information extraction tools, such as those described in co-pending Application Serial No. 10/033,823, filed Dec 19, 2001 and titled "Domain-Specific Knowledge-based MetaSearch System and Methods of Using" and co-pending Application Serial No. 10/641,492, both of which are incorporated herein, in their entireties, by reference thereto. '

Processing of data sets with respect to the ontology described with regard to Figs. 6A and 6B can be carried out in the same manner described above with regard to Fig. 5. In such manner, statistical scores may be assigned to the biological pathways 410 and subpathways 410 of the ontology, with regard to selected subsets of data sets that are the subject of interest. For example, Z-scores may be assigned to individual networks/subnetworks 410 based on a subset of up- and down-regulated genes from the gene data set 420 that may be from a gene expression experiment under a given condition. Networks/ontology terms 410 receiving statistically significant Z-scores may be identified as relevant/interesting networks. As with any other ontology, other scoring mechanisms, such as p-values may be used in addition to, or alternative to the Z-scoring. As in the previous example, such a scoring mechanism can be extended to proteins 440 and metabolite experimental data 460 as well, as long as information linking proteins and metabolites to the ontology 400 exists. For example, Z-scores may be assigned to identify interesting networks based on a set of over- or under-abundant proteins or metabolites as measured by different experimental techniques. The network analysis tool thus provides a mechanism to abstract the results from the data level (set of genes, proteins, or metabolites being relatively abundant or not) to the network level (for example, pathways or networks statistically found to be significantly differentially regulated).

The abstraction of results in terms of ontological term membership, from multiple sets of data, including diverse data sets, e.g., diverse experimental data sets studying a particular biological process or classification, allows for integration of results from these experimental studies. With the tools, systems and methods described, biologists/researchers can compare and contrast network models or other ontology terms that are statistically identified as significant from heterogeneous high-throughput experimental data studying genes, proteins, metabolites, other molecules and other high throughput data.

The results of scoring ontology terms for significance may be further computationally processed, as described above (e.g., determining significantly over or under represented ontology terms), given a selected subset of data. Additionally or alternatively, scoring results may be visualized, such as by using a tool as described co-pending Application Serial No. 10/403,762 or 10/688,588, for example. Aside from allowing a user to visually compare scoring results for any given ontology term with regard to multiple data types in side-by-side comparison, such a tool may further be employed to visually compare results between ontology terms, based upon multiple data types considered with respect to the ontology. For example, a term vector can be generated for each ontology term considered, wherein the term vector is constructed from the scores computed with respect to each subset of data considered with regard to that ontology term. There may be some benefit in using such term vectors to find ontology terms that receive similar scores across different data sets. Such ontology terms may signify certain biological process(es) that play a significant role in a process or phenomenon being experimentally studied. For comparison proposes, each vector should be constructed upon the same subsets of data, and in the same order. The term vectors can be used to identify ontological terms behaving similarly, where similar behavior may be defined as receiving similar significance scores, across the various experimental data sets. This may help identify other unknown relations among these ontological terms or their relation to the process being studied experimentally, since if two or more pathways score similarly over multiple data sets, it may be that they are related with respect to a biological process being studied, given that the data sets were generated as a result of such study. Such similarity may be a new discovery that may have gone previously undetected, given the added ability to extend similarity studies among data sets not directly related, through correlation using ontology terms as described.

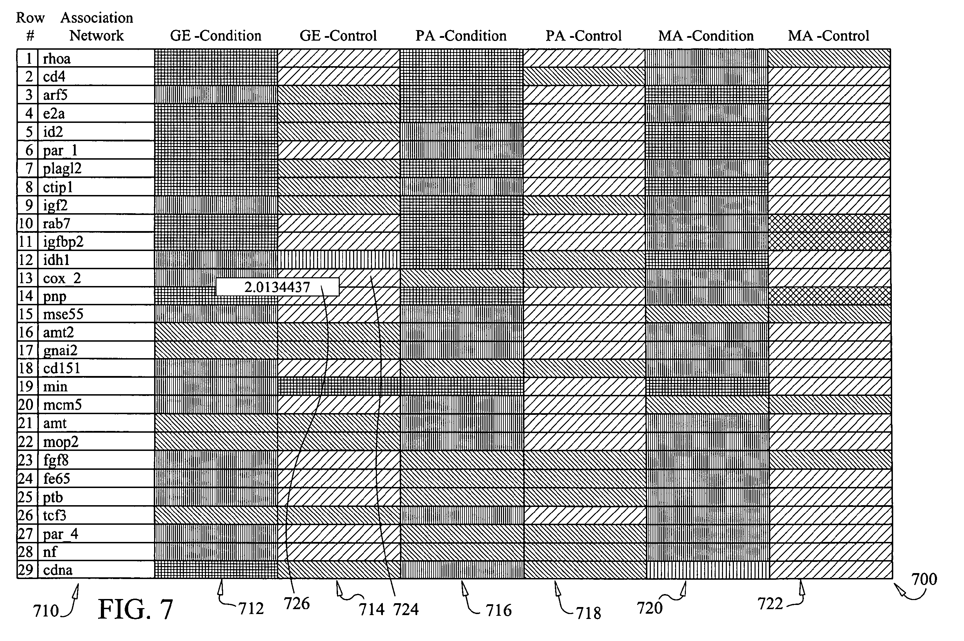

Fig. 7 shows an example of an output 700, produced on a display of a user interface (although such output may be printed or outputted using other known techniques), that may result from use of a tool as described in co-pending Application Serial No. 10/403,762 or 10/688,588, for example, in a manner as described. The abstraction of results in terms of ontology term membership, from sets of diverse experimental data studying a particular biological process or classification, allows for easy integration of results from these experimental studies. Using the techniques described, biologists/researchers can compare and contrast network models that are statistically identified as significant from diverse, high-throughput experimental data studying genes, proteins, and metabolites, for example, and constructing term vectors for further manipulation of the data to gain further insights.

In Fig. 7, a set of interesting pathways as determined by statistical scoring of the pathways (ontology terms) in a manner as described above, can be further identified as being interesting (or significantly overrepresented by members) with regard to each of the subsets of diverse data considered. For example, by loading the statistical results for each subset of diverse data with respect to each ontology term, a visualization 700 may be made to show the statistical results of at least a portion of the set of ontology terms (and which may be further compressible to show all of the ontology terms and results on a single display, or, is also scrollable to scroll through the entire set and display a selected portion).

Thus for example, in Fig. 7, the statistical results have been displayed for each of the sets of diverse data, with regard to whether a selected subset from the diverse set of data is or is not statistically overrepresented with regard to the ontology term being considered. More specifically, Fig. 7 displays the statistical results for gene expression data, both condition 712 and control 714 samples; protein abundance data, both condition 716 and control 718 samples; and metabolite abundance data, both condition 720 and control 722 samples with respect to each of the ontology terms (in this case, networks) 710 considered. The first column of number ("Row No.") simply displays an index of the row number for each row displayed. The numbers in this column are not rearranged during a row sort. The displayed results may be color-coded, as shown in Fig. 7, in a heat-map style presentation, where, in this example, those entries that are color-coded red, were values greater than a threshold value that indicated over-representation of that subset of data with respect to the ontology term being considered, and those entries color-coded green indicate values that are less than the threshold value, from which it can be concluded that the subset of data from the data set being considered was not statistically, significantly over-represented with respect to the ontology term being considered. As with heat maps, as the intensity of the color increases, so does the deviation from the threshold level. Thus, for example, a cell having a brighter or more intense red indicates a score that is greater than for a cell having a less bright or less red color. On the other hand, a brighter green cell represents a lower score (further below the threshold level) than a cell that is coded by a less bright green color. The display may further be provided with a tooltips feature 726 such that when a cursor is moved over a cell, or when a cell is selected by right mouse clicking, for example, then the numerical value of the cell that is represented by color coding is shown. For example, green cell 724 is further indicated to have a score of 2.0134437 in Fig. 7.

The cells for each of the experimental sets 712,714,716,718,720,722 for a single row combine to form a term vector that is representative of the relationships between each data set with the ontology term also located on that row. Thus, for each ontology term (in the example of Fig. 7, each network), a term vector may be constructed and visualized adjacent each term 710 (network name or designation). As noted above, the scores that are represented by the color-coded cells may be Z-scores, p-values, or other statistical indicators that indicate whether or not there is over-representation. Display 700 allows term vectors to be visualized adjacent one another for visual comparison as to similarities and differences. The results may be displayed in the order from which ontology terms are displayed in the ontology. Further, the rows (term vectors) may be similarity sorted according to any of the techniques taught in Application Serial Nos. 10/403,762 and 10/688,588. All ontology terms may be sorted with respect to a particular profile pattern. For example a sort according to a profile pattern showing high significance scores in the experimental conditions versus low significance scores in the control conditions may be carried out to find ontology terms that potentially differentiate the phenomenon (experimental condition) being studied. This may yield relations among the similarly scored ontology terms or between the ontology terms and the experimental condition being studied that may have been previously unknown.

The scores represented in the cells of display 700 were calculated with regard to a list of literature association networks 710 that were generated from the literature using methods described above. The six columns of data may be divided into two pairs, i.e., condition data 712,716,720 and control data 714,718,722. Each color-coded cell in the display represents the Z-score assigned to the specific network 710 in the same row that the cell is in, from subset data (e.g., interesting list of concepts, i.e., genes, proteins or metabolites) taken from the data set indicated by the column that the cell resides. High scoring term vectors (relative to scores of remaining term vectors characterizing the ontology being considered) may be further considered by a researcher as potentially linking diverse data types with respect to a phenomenon being studied. With further verification, the ontology terms identified by the present methods, systems and tool may be determined to provide a higher level link between diverse data types. Thus, for example, lists of genes, proteins and metabolites that significantly differentiate an experimental condition from the control may not be as meaningful to biologists as the ontology term "mitochondrion" or "fatty acid metabolism" which signifies that all these molecules are found in the mitochondrion or participate in fatty acid metabolism, respectively. The system thus aids in automatic identification of significant ontology terms form' lists of significant molecules.

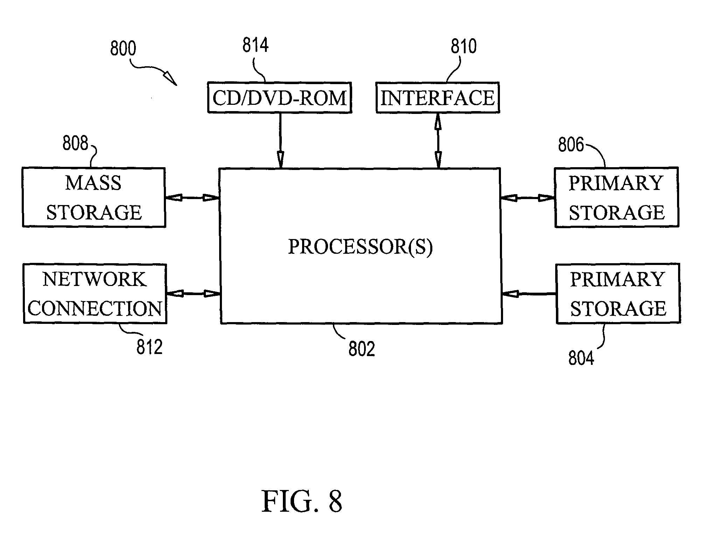

Fig. 8 illustrates a typical computer system that may be used to practice an embodiment of the present invention. The computer system 800 includes any number of processors 802 (also referred to as central processing units, or CPUs) that are coupled to storage devices including primary storage 806 (typically a random access memory, or RAM), primary storage 804 (typically a read only memory, or ROM). As is well known in the art, primary storage 804 acts to transfer data and instructions uni-directionally to the CPU and primary storage 806 is used typically to transfer data and instructions in a bidirectional manner Both of these primary storage devices may include any suitable computer-readable media such as those described above. A mass storage device 808 is also coupled bi-directionally to CPU 802 and provides additional data storage capacity and may include any of the computer-readable media described above. Mass storage device 808 may be used to store programs, data and the like and is typically a secondary storage medium such as a hard disk that is slower than primary storage. It will be appreciated that the information retained within the mass storage device 808, may, in appropriate cases, be incorporated in standard fashion as part of primary storage 806 as virtual memory. A specific mass storage device such as a CD-ROM or DVD-ROM 814 may also pass data uni-directionally to the CPU.

CPU 802 is also coupled to an interface 810 that includes one or more input/output devices such as such as video monitors, track balls, mice, keyboards, microphones, touch-sensitive displays, transducer card readers, magnetic or paper tape readers, tablets, styluses, voice or handwriting recognizers, or other well-known input devices such as, of course, other computers. CPU 802 optionally may be coupled to a computer or telecommunications network using a network connection as shown generally at 812. With such a network connection, it is contemplated that the CPU might receive information from the network, or might output information to the network in the course of performing the above-described method steps. The above-described devices and materials will be familiar to those of skill in the computer hardware and software arts.

The hardware elements described above may implement the instructions of multiple software modules for performing the operations of this invention. For example, instructions for calculating statistical significance may be stored on mass storage device 808 or 814 and executed on CPU 808 in conjunction with primary memory 806.

In addition, embodiments of the present invention further relate to computer readable media or computer program products that include program instructions and/or data (including data structures) for performing various computer-implemented operations. The media and program instructions may be those specially designed and constructed for the purposes of the present invention, or they may be of the kind well known and available to those having skill in the computer software arts. Examples of computer-readable media include, but are not limited to, magnetic media such as hard disks, floppy disks, and magnetic tape; optical media such as CD-ROM, CDRW, DVD-ROM, or DVD-RW disks; magneto-optical media such as floptical disks; and hardware devices that are specially configured to store and perform program instructions, such as read-only memory devices (ROM) and random access memory (RAM). Examples of program instructions include both machine code, such as produced by a compiler, and files containing higher level code that may be executed by the computer using an interpreter.

While the present invention has been described with reference to the specific embodiments thereof, it should be understood by those skilled in the art that various changes may be made and equivalents may be substituted without departing from the true scope of the invention. In addition, many modifications may be made to adapt a particular situation, material, composition of matter, process, process step or steps, to the objective, and scope of the present invention. All such modifications are intended to be within the scope of the claims appended hereto.

## Claims

1. A method of correlating data to higher level categories of characterization of the data, said method comprising:

   analyzing data from a first set of data (420,440,460) to determine where members of the first set map to an ontology (400);
   analyzing data from a second set of data (420,440,460) to determine where members of the second set map to the ontology (400);
   identifying a subset of the first set of data;
   identifying a subset of the second set of data;
   statistically analyzing the subset of the first set of data as it maps to the ontology (400) and identifying a first set of ontology terms (410) that are statistically differentiated by members of the subset of the first set of data;
   statistically analyzing the subset of the second set of data as it maps to the ontology (400) and identifying a second set of ontology terms (410) that are statistically differentiated by members of the subset of the second set of data; and
   correlating said first set of ontology terms (410) with said second set of ontology terms (410).

2. The method of claim 1, wherein said first set of ontology terms (410) are statistically overrepresented by said members of the subset of said first set of data (420,440,460), and said second set of ontology terms (410) are statistically overrepresented by said members of the subset of said second set of data (420,440,460).

3. The method of claim 1, wherein said first set of ontology terms (410) are statistically underrepresented by said members of the subset of said first set of data (420,440,460), and said second set of ontology terms (410) are statistically underrepresented by said members of the subset of said second set of data (420,440,460).

4. The method of any of claims 1-3, wherein said first and second sets of data (420,440,460) contain disparate data types relative to one another.

5. The method of any of claims 1-4, further comprising identifying members of said subset of the first set of data (420,440,460), and members of said subset of the second set of data (420,440,460) that map to ontology terms (410) that have been correlated.

6. The method of any of claims 1-5, wherein the first set of data (420,440,460) is generated from at least one control sample and at least one experimental sample and the subset of the first set of data contains data that differentiates a measured characteristic of said at least one experimental sample from said at least one control sample; and wherein the second set of data (420,440,460) is generated from said at least one control sample and said at least one experimental sample, and the subset of the second set of data contains data that differentiates another measured characteristic of said at least one experimental sample form said at least one control sample.

7. The method of claim 6, wherein said identifying a subset of the first set of data comprises identifying the subset of members of said first set of data that differentiate said measured characteristic of said at least one experimental sample from at least said one control sample the greatest; and wherein said identifying a subset of the second set of data comprises identifying the subset of members of said second set of data that differentiate said measured characteristic of said at least one experimental sample from said at least one control sample the greatest.

8. The method of any of claims 1-7, wherein said first set of data (420,440,460) is biological data and said second set of data is biological data (420,440,460).

9. The method of any of claims 1-8, wherein said first and second sets of data (420,440,460) are independent of one another, but derived from the same biological samples.

10. The method of any of claims 1-9, wherein statistical differentiation is calculated based on a threshold value, said method further comprising altering said threshold value and repeating the steps of claim 1.

11. The method of any of claims 1-5 and 8-10, wherein the first set of data (420,440,460) is generated from at least one control sample and at least one experimental sample and the subset of the first set of data is selected based upon a predetermined profile of data values relative to said at least one control sample and at least one experimental sample; and
wherein the second set of data (420,440,460) is generated from said at least one control sample and said at least one experimental sample, and the subset of the second set of data is selected based upon a second predetermined profile of data values relative to said at least one control sample and at least one experimental sample.

12. The method of claim 11, wherein said predetermined profile is the same as said second predetermined profile.

13. The method of claim 11, wherein said predetermined profile is different from said second predetermined profile.

14. The method of any of claims 1-13, further comprising
analyzing data from at least one additional set of data (420,440,460) to determine where members of each said additional set map to the ontology (400);
identifying a subset of each said additional set of data (420,440,460);
statistically analyzing each said subset of each additional set of data (420,440,460) as each maps to the ontology (400) and, for each additional set, identifying a set of ontology terms (410) that are statistically over-represented by members of the subset of that additional first set of data (420,440,460), respectively; and
correlating each said set of ontology terms (410) identified with respect to each said additional set of data (420,440,460), with said first and second sets of ontology terms (410).

15. The method of any of claims 1-14, further comprising generating a term vector from results regarding each ontology term (4100 considered, respectively; and comparing said term vectors.

16. The method of any of claims 1-15, further comprising visually displaying results of said correlating.

17. The method of claim 15 or claim 16, further comprising visually displaying results of said generating term vectors.

18. The method of claim 16 or claim 17, further comprising sorting said results based on interactive user input.

19. The method of claim 18, wherein said sorting comprises at least one of similarity sorting and sorting with respect to a predetermined profile pattern.

20. The method of claim 18 or 19, further comprising selecting a subset of the sorted term vectors based upon a threshold value for similarity with respect to said predetermined profiled pattern.

21. The method of claim 20, further comprising displaying said subset of the sorted term vectors as ontology terms (410) that have been determined to be significant regarding the correlation of the data.

22. A system for correlating data from data sets to higher level categories of characterization of the data, said system comprising:

   means for analyzing data from a first set of data (420,440,460) to determine where members of the first set map to an ontology (410);
   means for analyzing data from a second set of data (420,440,460) to determine where members of the second set map to the ontology (410);

means for identifying a subset of the first set of data (420,440,460); ,

means for identifying a subset of the second set of data (420,440,460);

means for statistically analyzing the subset of the first set of data (420,440,460) as it maps to the ontology (400) and identifying a first set of ontology terms (410) that are statistically differentiated by members of the subset of the first set of data (420,440,460);

means for statistically analyzing the subset of the second set of data (420,440,460) as it maps to the ontology (400) and identifying a second set of ontology terms (410) that are statistically differentiated by members of the subset of the second set of data (420,440,460); and

means for correlating said first set of ontology terms (410) with said second set of ontology terms (410).

23. The system of claim 22, further comprising a user interface configured for user interaction with processing by said system.

24. A computer readable medium carrying one or more sequences of instructions for correlating data from data sets to higher level categories of characterization of the data, wherein execution of one or more sequences of instructions by one or more processors causes the one or more processors to perform the steps of:

analyzing data from a first set of data (420,440,460) to determine where members of the first set map to an ontology (400);

analyzing data from a second set of data (420,440,460) to determine where members of the second set map to the ontology (400);

identifying a subset of the first set of data (420,440,460); >

identifying a subset of the second set of data (420,440,460);

statistically analyzing the subset of the first set of data (420,440,460) as it maps to the ontology (400) and identifying a first set of ontology terms (410) that are statistically differentiated by members of the subset of the first set of data (420,440,460);

analyzing the subset of the second set of data (420,440,460) as it maps to the ontology (400) and identifying a second set of ontology terms (410) that are statistically differentiated by members of the subset of the second set of data (420,440,460); and

correlating said first set of ontology terms (410) with said second set of ontology terms (410.

FIG. 1

FIG. 2

FIG. 3

FIG. 4B

420

g₁
g₂
g₃ ... g₅₀₀

FIG. 4C

440

p₁
p₂
p₃ ... p₅₀₀

FIG. 4D

460

m₁
m₂
m₃ ... m₅₀₀

400

410 structural constituent for ribosome

410 structural molecular activity
$g_{27}, g_{411}$
$p_{27}, p_{411}$

410 receptor binding
$g_{111}, g_{155}, g_{240}, g_{360}$
$p_{111}, p_{156}, p_{157}, p_{240}, p_{360}$

410 signal transducer activity
$g_1, g_5, g_{313}$
$p_1, p_5, p_{313}$
$m_7, m_9, m_{145}$

410 protein binding
$g_{53}, g_{211}, g_{333}$
$p_{53}, p_{211}, p_{333}$
$m_{27}, m_{55}, m_{246}, m_{300}$

410 binding

410 molecular function

FIG. 4A

Provide at least first and second sets
of data of different data types — 510

Select a subset from each set — 512

For each ontology term, count the occurrences
of data from each subset that map to that term — 514

For each ontology term and for each subset,
determine the significance of over/under
abundance of subset members in the ontology term — 516

Compare significance scores for ontology term
across subset scores with respect to that ontology term — 518

Statistical scoring of ontology terms as to
degree to which data sets are linked — 520

FIG. 5

EP 1 647 912 A2

400

410
Pathway 1

410
Subpathway 1,1

Subpathway 1,2

410

410
Pathway 2

410
Subpathway 2,1

Subpathway 2,2

Subpathway 2,2,1

410

410

410
Pathway 3

Subpathway 3,1

410

410

410
Pathway 4

FIG. 6A

FIG. 6B

| Row # | Association Network | GE -Condition | GE -Control | PA -Condition | PA -Control | MA -Condition | MA -Control |
|---|---|---|---|---|---|---|---|
| 1 | rhoa | | | | | | |
| 2 | cd4 | | | | | | |
| 3 | arf5 | | | | | | |
| 4 | e2a | | | | | | |
| 5 | id2 | | | | | | |
| 6 | par_1 | | | | | | |
| 7 | plagl2 | | | | | | |
| 8 | ctip1 | | | | | | |
| 9 | igf2 | | | | | | |
| 10 | rab7 | | | | | | |
| 11 | igfbp2 | | | | | | |
| 12 | idh1 | | | | | | |
| 13 | cox_2 | | | | | | |
| 14 | pnp | 2.0134437 | | | | | |
| 15 | mse55 | | | | | | |
| 16 | amt2 | | | | | | |
| 17 | gnai2 | | | | | | |
| 18 | cd151 | | | | | | |
| 19 | min | | | | | | |
| 20 | mcm5 | | | | | | |
| 21 | amt | | | | | | |
| 22 | mop2 | | | | | | |
| 23 | fgf8 | | | | | | |
| 24 | fe65 | | | | | | |
| 25 | ptb | | | | | | |
| 26 | tcf3 | | | | | | |
| 27 | par_4 | | | | | | |
| 28 | nf | | | | | | |
| 29 | cdna | | | | | | |

710    FIG. 7    712   726   714   724    716    718    720    722    700

FIG. 8